(19) Europäisches Patentamt
European Patent Office
Office européen des brevets

(11) **EP 4 324 786 A1**

(12) **EUROPÄISCHE PATENTANMELDUNG**

(43) Veröffentlichungstag:
**21.02.2024 Patentblatt 2024/08**

(21) Anmeldenummer: **22190457.6**

(22) Anmeldetag: **16.08.2022**

(51) Internationale Patentklassifikation (IPC):
***C01B 3/36*** *(2006.01)* ***C01B 3/38*** *(2006.01)*
***C07C 29/151*** *(2006.01)* ***C25B 1/04*** *(2021.01)*

(52) Gemeinsame Patentklassifikation (CPC):
(C-Sets verfügbar)
**C01B 3/382; C01B 3/36; C07C 29/1518; C25B 1/04; C25B 15/08;** C01B 2203/0244; C01B 2203/0255; C01B 2203/061; C01B 2203/1241

(Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **L'AIR LIQUIDE, SOCIETE ANONYME POUR L'ETUDE ET L'EXPLOITATION DES PROCEDES GEORGES CLAUDE**
**75007 Paris (FR)**

(72) Erfinder:
- **Gronemann, Veronika**
  **60439 Frankfurt am Main (DE)**
- **Oelmann, Tobias**
  **60439 Frankfurt am Main (DE)**
- **Williams, Bryce**
  **60388 Frankfurt am Main (DE)**

(74) Vertreter: **Stang, Stefan**
**Air Liquide Forschung und Entwicklung GmbH**
**Gwinnerstraße 27-33**
**60388 Frankfurt am Main (DE)**

(54) **VERFAHREN UND ANLAGE ZUR BEREITSTELLUNG VON SYNTHESEGAS UND ZUR HERSTELLUNG VON METHANOL**

(57) Erfindungsgemäß wird ein Verfahren und eine Anlage zur Produktion eines Synthesegasstroms angegeben, welches es ermöglicht den Synthesegasstrom mit einer für die Methanolsynthese oder anderer Synthesen geeigneten Stöchiometriezahl zu erzeugen. Dafür wird einem kohlenwasserstoffhaltigen Einsatzgasstrom ein elektrolytisch erzeugter Wasserstoffstrom zugemischt, und der resultierende wasserstoffhaltige und kohlenwasserstoffhaltige Einsatzgasstrom wird in einem Reformierungsschritt unter Beteiligung von Sauerstoff zu Synthesegas umgesetzt. Die Verfahrensführung gemäß Erfindung hat den Vorteil, dass der elektrolytisch erzeugte Wasserstoffstrom nicht aufbereitet werden muss, insbesondere kein Sauerstoff aus dem elektrolytisch erzeugten Wasserstoffstrom entfernt werden muss. Die Erfindung umfasst ferner ein Verfahren und eine Anlage zur Produktion von Methanol, aufweisend das erfindungsgemäße Verfahren und die erfindungsgemäße Anlage zur Produktion des Synthesegasstroms.

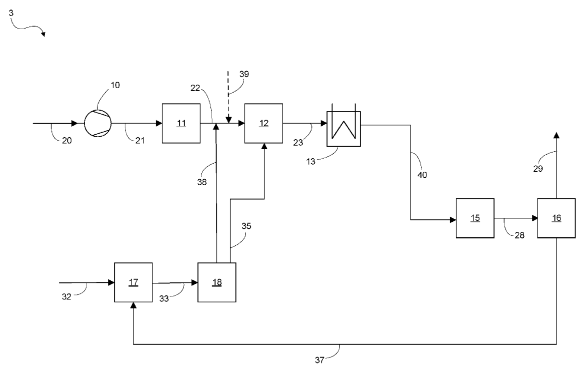


Fig. 3

EP 4 324 786 A1

(52) Gemeinsame Patentklassifikation (CPC): (Forts.)

C-Sets
**C07C 29/1518, C07C 31/04**

## Beschreibung

### Gebiet der Erfindung

Die Erfindung betrifft ein Verfahren und eine Anlage zum Herstellen von Synthesegas. Die Erfindung betrifft ferner ein Verfahren zum Herstellen von Methanol, umfassend vorgenanntes Verfahren zum Herstellen von Synthesegas. Die Erfindung betrifft ferner eine Methanolanlage zum Herstellen von Methanol, umfassend vorgenannte Anlage zum Herstellen von Synthesegas.

### Stand der Technik

Methanol wird im großtechnischen Maßstab aus Synthesegas hergestellt. Synthesegas ist ein Gemisch aus vorwiegend Wasserstoff ($H_2$), Kohlenmonoxid (CO) sowie Kohlendioxid ($CO_2$). Kohlenmonoxid sowie Kohlendioxid werden dabei häufig unter dem Begriff "Kohlenoxide" oder "Kohlenstoffoxide" zusammengefasst. Dabei laufen an einem festen Methanolsynthese-Katalysator unter anderem die beiden folgenden Gleichgewichtsreaktionen (1) und (2) nebeneinander ab.

$$(1)\qquad CO_2 + 3\,H_2 \rightleftharpoons CH_3OH + H_2O$$

$$(2)\qquad CO + 2\,H_2 \rightleftharpoons CH_3OH$$

Die Zusammensetzung des Synthesegases ist durch die sogenannte Stöchiometriezahl SN, definiert als

$$SN = \frac{n(H_2) - n(CO_2)}{n(CO) + n(CO_2)}\,, mit\ n\ in\ [mol],$$

charakterisiert. Eine für die Methanolsynthese stöchiometrisch ausbilanzierte Synthesegaszusammensetzung ist durch eine Stöchiometriezahl SN von 2,0 gekennzeichnet. Werte von kleiner als 2,0 weisen auf ein Wasserstoffdefizit hin, während Werte von größer als 2,0 auf einen Wasserstoffüberschuss hinweisen. Bei einem Synthesegas mit einer Stöchiometriezahl von kleiner als 2,0 wird auch von einem unterstöchiometrischen oder substöchiometrischen Synthesegas gesprochen.

Synthesegase mit Wasserstoffdefizit werden regelmäßig bei Prozessen erhalten, welche einen partiellen Oxidationsschritt umfassen. Dazu gehören partielle Oxidation (POX) als solche und autotherme Reformierung (ATR).

Die Hauptelemente eines ATR-Reaktors sind ein Brenner, eine Brennkammer und ein Katalysatorbett in einem feuerfest ausgekleideten Druckmantel. In einem ATR-Reaktor folgt auf die partielle Oxidation eines kohlenwasserstoffhaltigen Einsatzstroms durch unterstöchiometrische Mengen von Sauerstoff eine Dampfreformierung des teilweise oxidierten kohlenwasserstoffhaltigen Einsatzstroms an einem Festbett aus Dampfreformierungskatalysator.

Eine partielle Oxidation läuft für Methan als Kohlenwasserstoff vor allem nach folgender Reaktionsgleichung (3) ab.

$$(3)\qquad CH_4 + 1\tfrac{1}{2}\,O_2 \rightleftharpoons CO + 2\,H_2O$$

Die exotherme partielle Oxidation liefert somit Dampf und die erforderliche Wärmeenergie für die endotherme Dampfreformierungsreaktion, welche nach Reaktionsgleichung (4) abläuft.

$$(4)\qquad CH_4 + H_2O \rightleftharpoons 3\,H_2 + CO$$

Aufgrund der hohen Temperatur findet die Dampfreformierung (4) bis zu einem gewissen Grad auch in der Brennkammer des Reaktors ohne Beteiligung des Katalysators statt.

Die Dampfreformierungsreaktion wird von der Wassergas-Shift-Reaktion gemäß Reaktionsgleichung (5) begleitet, welche ebenfalls exotherm verläuft.

$$(5) \quad CO + H_2O \rightleftharpoons CO_2 + H_2$$

Normalerweise befindet sich das Gas am Ausgang des ATR-Reaktors in Bezug auf die Dampfreformierung und die Wassergas-Shift-Reaktion im oder nahe am thermodynamischen Gleichgewicht.

Bei einer ATR wird dem eingesetzten Kohlenwasserstoff häufig Dampf zugesetzt. Die genaue Zusammensetzung des Synthesegases am Ausgang des Reaktors hängt vom Verhältnis Kohlenwasserstoff zu Dampf des Einsatzstroms, der Prozesstemperatur und dem Prozessdruck ab. Die Temperatur des Gasgemischs am Ausgang des ATR-Reaktors liegt typischerweise im Bereich von 950 bis 1050 Grad Celsius. Der Prozessdruck liegt typischerweise bei 30 bis 80 bar.

Eine Alternative zur Herstellung von Synthesegas ist die partielle Oxidation als solche, auch als POX bezeichnet. Die Hauptelemente eines POX-Reaktors sind ein Brenner und eine Brennkammer, die sich in einem feuerfest ausgekleideten Druckmantel befinden. In einem POX-Reaktor findet eine partielle Oxidation eines kohlenwasserstoffhaltigen Einsatzstroms durch unterstöchiometrische Mengen Sauerstoff statt. Es findet auch eine gewisse (nicht katalysierte) Dampfreformierung statt und die Wassergas-Shift-Reaktion begleitet die partielle Oxidation. Die obigen Reaktionsgleichungen (3) bis (5) sind somit ebenfalls anwendbar. Die Temperatur des Gasgemisches am Ausgang des Reaktors liegt typischerweise im Bereich von 1250 bis 1450 Grad Celsius. Der Prozessdruck liegt typischerweise bei 30 bis 100 bar.

Wie bereits erwähnt liefern die beiden oben beschriebenen Prozesse üblicherweise ein deutlich unterstöchiometrisches Synthesegas, also ein Synthesegas mit einer Stöchiometriezahl von deutlich kleiner als 2,0. Ein solches Synthesegas weist in Bezug auf die Synthese von Methanol ein Wasserstoff-Defizit auf. Wird ein solches Synthesegas direkt für die Herstellung von Methanol verwendet, so wird der Wasserstoff somit nahezu vollständig verbraucht, während ein wesentlicher Teil der Kohlenstoffoxide nicht umgesetzt wird. Dies hat unter anderem zur Folge, dass der Gehalt an Nebenprodukten (insbesondere höhere Alkohole und Ketone) höher ist als erwünscht und dass die Ausbeute an Methanol nicht optimal ist.

Aus diesem Grund wird in einem industriellen Prozess dem Strom des unterstöchiometrischen Synthesegases in der Regel ein Strom reinen Wasserstoffs zugeführt, um die Stöchiometriezahl SN des Synthesegases auf zumindest 1,9 zu erhöhen, vorzugsweise auf 2,0 oder mehr zu erhöhen.

Dafür wird in konventionellen Verfahren ein Teil des reformierten Synthesegases vom Hauptstrom abgetrennt und Wasserstoff aus diesem Teilstrom in der Regel durch Druckwechseladsorption *(pressure swing adsorption, PSA)* abgetrennt. Das bei der Druckwechseladsorption anfallende Abgas oder *tail gas* enthält Kohlenwasserstoffe und/oder Kohlenmonoxid und kann daher als Brenngas für den ATR- oder POX-Reaktor verwertet werden.

Wird der Wasserstoff stattdessen durch einen Elektrolyseur bereitgestellt, kann ein Teil des für die Methanolsynthese erforderlichen Synthesegases eingespart werden, wodurch sich der $CO_2$-Fußabdruck der Anlage verbessert.

Die EP 3 658 494 B1 schlägt daher vor, Wasserstoff aus einem Elektrolyseur einem Synthesegas-Strom aus einem POX- oder ATR-Reaktor beizumischen. Zusätzlich wird Sauerstoff aus einer Luftzerlegungseinheit als Oxidationsmittel zum Betrieb des Brenners des POX- oder ATR-Reaktors verwendet.

Der von einem Elektrolyseur bereitgestellte Wasserstoff enthält üblicherweise Reste von bis zu 1 Vol.-% Sauerstoff, oder mehr. Dies hängt mit nicht zu vermeidenden Diffusionsprozessen zusammen, welche innerhalb einer Elektrolysezelle zwischen dem Anoden- und Kathodenraum ablaufen. Unter anderem diffundiert stets eine gewisse Menge des anodisch erzeugten Sauerstoffs durch die Membran oder das Diaphragma vom Anoden- in den Kathodenraum und verunreinigt dadurch den kathodisch erzeugten Wasserstoff.

Sauerstoff stellt für Methanol-Katalysatoren ein Katalysatorgift dar und muss daher möglichst quantitativ aus dem kathodisch erzeugten Wasserstoffstrom entfernt werden, bevor dieser dem Synthesegas-Strom aus der POX- oder ATR-Einheit beigemischt wird. Dies erfolgt in einer sogenannten De-Ox Einheit. Diese umfasst eine katalytische Stufe zur quantitativen Umsetzung des verunreinigenden Sauerstoffs mit Wasserstoff des Wasserstoffstroms zu Wasser. Das katalytisch so erzeugte Wasser wird anschließend adsorptiv gebunden, beispielsweise an einem Molekularsieb. Dadurch wird ein sauerstoff- und wasserfreier Wasserstoffstrom erhalten.

## Beschreibung der Erfindung

Eine allgemeine Aufgabe der vorliegenden Erfindung besteht darin ein Verfahren und eine Anlage zur Bereitstellung eines Synthesegases vorzuschlagen, welche(s) die Nachteile des Standes der Technik zumindest teilweise überwindet.

Eine Aufgabe der vorliegenden Erfindung besteht darin ein Verfahren und eine Anlage zur Bereitstellung eines Synthesegases vorzuschlagen, wobei das bereitgestellte Synthesegas so zusammengesetzt ist, dass es sich unmittelbar für die Herstellung von Methanol an einem Methanolsynthese-Katalysator eignet.

Eine Aufgabe der vorliegenden Erfindung besteht darin ein Verfahren und eine Anlage zur Bereitstellung eines Synthesegases vorzuschlagen, wobei die Einstellung der Stöchiometriezahl des Synthesegases durch Zugabe von

elektrolytisch erzeugtem Wasserstoff erfolgt, und wobei der dafür eingesetzte elektrolytisch erzeugte Wasserstoff keinem Verfahren zur Entfernung von Sauerstoff unterzogen werden muss.

Eine Aufgabe der vorliegenden Erfindung besteht darin ein Verfahren und eine Anlage zur Bereitstellung eines Synthesegases mit einer Stöchiometriezahl von 1,9 oder größer vorzuschlagen, wobei die Einstellung der Stöchiometriezahl durch Zugabe von elektrolytisch erzeugtem Wasserstoff erfolgt, und wobei der dafür eingesetzte elektrolytisch erzeugte Wasserstoff keinem Verfahren zur Entfernung von Sauerstoff unterzogen werden muss.

Eine Aufgabe der vorliegenden Erfindung besteht ferner darin ein Verfahren und eine Anlage zur Herstellung von Methanol vorzuschlagen, welche(s) die vorgenannten Aufgaben zumindest teilweise löst.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie sind, soweit zutreffend, ebenso bevorzugt für gleichnamige oder entsprechende Bestandteile einer jeweils anderen erfindungsgemäßen Kategorie. Die Ausdrücke "aufweisend", "umfassend" oder "beinhaltend" etc. schließen nicht aus, dass weitere Elemente, Inhaltsstoffe etc. enthalten sein können. Der unbestimmte Artikel "ein" schließt nicht aus, dass eine Mehrzahl vorhanden sein kann.

Die vorgenannten Aufgaben werden zumindest teilweise gelöst durch ein Verfahren zum Herstellen von Synthesegas, insbesondere von Synthesegas für die Methanolsynthese, umfassend die Schritte:

(a) Bereitstellen eines kohlenwasserstoffhaltigen Einsatzgasstroms;
(b) Bereitstellen eines elektrolytisch erzeugten Wasserstoffstroms;
(c) Zuführen zumindest eines Teils des elektrolytisch erzeugten Wasserstoffstroms zu dem kohlenwasserstoffhaltigen Einsatzgasstrom, wodurch ein wasserstoffhaltiger Einsatzgasstrom erhalten wird;
(d) Umsetzen des wasserstoffhaltigen Einsatzgasstroms unter Beteiligung von Sauerstoff als Oxidationsmittel in einem Reformierungsschritt zu einem Synthesegasstrom.

Die Schritte (a) bis (d) werden nicht zwangsläufig in der vorgegebenen Reihenfolge durchgeführt.

Erfindungsgemäß wird der elektrolytisch erzeugte Wasserstoffstrom dem kohlenwasserstoffhaltigen Einsatzgasstrom gemäß Schritt (c) vor der Umsetzung des Einsatzgasstroms zu Synthesegas gemäß Schritt (d) zugeführt. Der elektrolytisch erzeugte Wasserstoffstrom wird dem kohlenwasserstoffhaltigen Einsatzgasstrom insbesondere stromaufwärts des jeweiligen für den Reformierungsschritt konfigurierten Reaktor zugeführt. Bei diesem Reaktor handelt es sich insbesondere um einen POX- oder einen ATR-Reaktor. Der so erhaltene wasserstoffhaltige Einsatzgasstrom wird dann gemäß Schritt (d) unter Beteiligung von Sauerstoff als Oxidationsmittel zu Synthesegas umgesetzt.

Es ist daher erfindungsgemäß möglich, einen sauerstoffhaltigen elektrolytisch erzeugten Wasserstoffstrom für die Umsetzung gemäß Schritt (d) zu verwenden.

Dabei handelt es sich bei dem Reformierungsschritt gemäß Schritt (d) zwangsläufig um einen Reformierungsschritt, bei welchem Sauerstoff im Verhältnis zu dem eingesetzten Kohlenwasserstoff in unterstöchiometrischer Menge eingesetzt wird. Wäre dies nicht der Fall, so könnte kein Synthesegasstrom aus dem wasserstoffhaltigen Einsatzgasstrom erzeugt werden. Der eingesetzte Sauerstoff wird gemäß Schritt (d) somit vollständig verbraucht, so dass der erzeugte Synthesegasstrom frei von Sauerstoff ist.

Würde der elektrolytisch erzeugte Wasserstoffstrom dem Synthesegasstrom gemäß Stand der Technik erst nach der Umsetzung gemäß Schritt (d) zugeführt, so erforderte dies eine quantitative Entfernung von Sauerstoff aus dem elektrolytisch erzeugten Wasserstoffstrom. Wie oben beschrieben stellt Sauerstoff für Methanolsynthese-Katalysatoren und andere Katalysatorarten ein Katalysatorgift dar.

Der gemäß Schritt (c) erzeugte wasserstoffhaltige Einsatzgasstrom setzt sich aus dem kohlenwasserstoffhaltigen Einsatzgasstrom und dem elektrolytisch erzeugten Wasserstoffstrom zusammen. Bei dem "wasserstoffhaltigen Einsatzgasstrom" handelt es sich somit um einen wasserstoffhaltigen und kohlenwasserstoffhaltigen Einsatzgasstrom.

Der elektrolytisch erzeugte Wasserstoffstrom wird durch einen Elektrolyseur bereitgestellt. Je nach Verfahrensführung wird ein Teil des so elektrolytisch erzeugten Wasserstoffstroms dem kohlenwasserstoffhaltigen Einsatzgasstrom zugeführt oder der vollständige so elektrolytisch erzeugte Wasserstoffstrom dem kohlenwasserstoffhaltigen Einsatzgasstrom zugeführt. Vorzugsweise wird der Elektrolyseur so ausgelegt, dass der vollständige elektrolytisch erzeugte Wasserstoffstrom dem kohlenwasserstoffhaltigen Einsatzgasstrom zugeführt wird. Der Elektrolyseur kann dann die meiste Zeit unter Volllast betrieben werden. Der Elektrolyseur wird dann vorzugsweise so ausgelegt, dass der resultierende aus dem wasserstoffhaltigen Einsatzgasstrom erzeugte Synthesegasstrom eine für die Methanolsynthese geeignete Stöchiometriezahl aufweist.

Der elektrolytisch erzeugte Wasserstoffstrom kann durch jedes dem Fachmann geläufige Elektrolyse-Verfahren erzeugt werden. Vorzugsweise handelt es sich bei dem Elektrolyse-Verfahren um eine Wasserelektrolyse. Beispiele sind alkalische Elektrolyse, Protonenaustauschermembran-Elektrolyse (PEM-Elektrolyse), Anionenaustauschermembran-Elektrolyse (AEM-Elektrolyse), Hochtemperaturelektrolyse (HTE) und Elektrolyse unter Verwendung von Festoxid-Elektrolyseurzellen (SOEC).

Der kohlenwasserstoffhaltige Einsatzgasstrom weist vorzugsweise Methan ($CH_4$) als Hauptkomponente auf. Der kohlenwasserstoffhaltige Einsatzgasstrom weist vorzugsweise mindestens 50 Vol.-% Methan auf, oder mindestens 75 Vol.-% Methan, oder mindestens 90 Vol.-% Methan, oder mindestens 95 Vol.-% Methan.

Der Synthesegasstrom weist Synthesegas auf. Ein Synthesegas ist ein Gasgemisch, welches zumindest ein Kohlenstoffoxid (Kohlenmonoxid oder Kohlendioxid) und Wasserstoff aufweist. Vorzugsweise weist das Synthesegas Kohlenmonoxid, Kohlendioxid und Wasserstoff auf.

Unter einem Reformierungsschritt wird grundsätzlich die chemische Umsetzung von Kohlenwasserstoffen mit Sauerstoff und/oder Dampf zu einem Synthesegas verstanden.

Eine bevorzugte Ausführungsform des erfindungsgemäßen Verfahrens ist dadurch gekennzeichnet, dass der Reformierungsschritt ein autothermes Reformieren (ATR) oder eine partielle Oxidation (POX) des wasserstoffhaltigen Einsatzgasstroms umfasst.

Eine bevorzugte Ausführungsform des erfindungsgemäßen Verfahrens ist dadurch gekennzeichnet, dass eine gemäß Schritt (c) zum kohlenwasserstoffhaltigen Einsatzgasstrom zugeführte Menge an elektrolytisch erzeugtem Wasserstoffstrom so eingestellt wird, dass gemäß Schritt (d) ein Synthesegasstrom erhalten wird, welcher eine Stöchiometriezahl SN von 1,9 bis 2,5 aufweist, vorzugsweise von 2,0 bis 2,4 aufweist, wobei gilt

$$SN = \frac{n(H_2) - n(CO_2)}{n(CO) + n(CO_2)}, mit\ n\ in\ [mol].$$

Dadurch wird ein Synthesegasstrom erhalten welcher direkt, also unmittelbar für eine nachgeschaltete Methanolsynthese, verwendet werden kann. Zumindest kann der so erhaltene Synthesegasstrom für eine nachgeschaltete Methanolsynthese verwendet werden, ohne dass eine weitere Zuführung von Wasserstoff erforderlich ist. Der so erhaltene Synthesegasstrom ist ebenso für andere Synthesen unmittelbar oder zumindest ohne weitere Zuführung von Wasserstoff einsetzbar, welche Synthesegaszusammensetzungen mit entsprechender Stöchiometriezahl erfordern.

Eine bevorzugte Ausführungsform des erfindungsgemäßen Verfahrens ist dadurch gekennzeichnet, dass der elektrolytisch erzeugte Wasserstoffstrom als Verunreinigung Sauerstoff enthält, und der als Verunreinigung enthaltene Sauerstoff vor dem Zuführen des elektrolytisch erzeugten Wasserstoffstroms zu dem kohlenwasserstoffhaltigen Einsatzgasstrom gemäß Schritt (c) nicht entfernt wird.

Wie bereits erläutert ist es erfindungsgemäß nicht erforderlich, als Verunreinigung im elektrolytisch erzeugten Wasserstoffstrom enthaltenen Sauerstoff durch ein aufwändiges Reinigungsverfahren zu entfernen. Der als Verunreinigung im elektrolytisch erzeugten Wasserstoffstrom enthaltene Sauerstoff wird im Reformierungsschritt (d) als Oxidationsmittel vollständig verbraucht.

Eine weitere bevorzugte Ausführungsform des erfindungsgemäßen Verfahrens ist dadurch gekennzeichnet, dass der elektrolytisch erzeugte Wasserstoffstrom Wasser enthält, und das Wasser vor dem Zuführen des elektrolytisch erzeugten Wasserstoffstroms zu dem kohlenwasserstoffhaltigen Einsatzgasstrom gemäß Schritt (c) nicht entfernt wird.

Da im Reformierungsschritt gemäß Schritt (d) zumindest teilweise auch eine Dampfreformierung abläuft und Wasserdampf so verbraucht wird, ist es auch nicht erforderlich, den elektrolytisch erzeugten Wasserstoffstrom zu trocknen. Ein elektrolytisch erzeugter Wasserstoffstrom enthält ohne weitere Aufarbeitung stets gewisse Mengen an Restwasser, da die Trennung im Gas-Flüssigkeits-Separator eines Elektrolyseurs oder einer vergleichbaren Vorrichtung niemals vollständig ist.

Der elektrolytisch erzeugte Wasserstoffstrom kann als Verunreinigung bis zu 5 Vol.-% Sauerstoff enthalten, oder 0,01 bis 5 Vol.-% Sauerstoff enthalten, oder 0,1 bis 3 Vol.-% Sauerstoff enthalten, oder 0,1 bis 1 Vol.-% Sauerstoff enthalten.

Eine bevorzugte Ausführungsform des erfindungsgemäßen Verfahrens ist dadurch gekennzeichnet, dass das Verfahren das Bereitstellen eines elektrolytisch erzeugten Sauerstoffstroms umfasst, wobei der elektrolytisch erzeugte Sauerstoffstrom als Oxidationsmittel in Schritt (d) verwendet wird.

Grundsätzlich verläuft der Reformierungsschritt gemäß Schritt (d) unter Beteiligung von Sauerstoff als Oxidationsmittel. Dabei kann Luft zum Einsatz kommen, oder mit Sauerstoff angereicherte Luft zum Einsatz kommen, oder reiner Sauerstoff zum Einsatz kommen. Insbesondere wird das Oxidationsmittel dem Brenner einer POX- oder ATR-Einheit zugeführt und das wasserstoffhaltige und kohlenwasserstoffhaltige Einsatzgas dort mit einer unterstöchiometrischen Menge Sauerstoff zu Synthesegas umgesetzt.

Ein Elektrolyseur erzeugt üblicherweise Sauerstoff als "Nebenprodukt", wobei dieses Nebenprodukt häufig nicht verwertet wird. Die Verwendung des elektrolytisch erzeugten Sauerstoffs als Oxidationsmittel für den Reformierungsschritt gemäß Schritt (d) stellt somit eine Verbesserung der Prozessintegration des erfindungsgemäßen Verfahrens dar.

Alternativ dazu umfasst das Verfahren das Bereitstellen eines durch Luftzerlegung erzeugten Sauerstoffstroms,

wobei der durch Luftzerlegung erzeugte Sauerstoffstrom als Oxidationsmittel in Schritt (d) verwendet wird.

Alternativ dazu kann der Sauerstoff dem Prozess auch über eine pipeline zur Verfügung gestellt werden.

Eine bevorzugte Ausführungsform des erfindungsgemäßen Verfahrens ist dadurch gekennzeichnet, dass dem kohlenwasserstoffhaltigen Einsatzgasstrom oder dem wasserstoffhaltigen Einsatzgasstrom ein Dampfstrom zugeführt wird.

Optional wird dem kohlenwasserstoffhaltigen Einsatzgasstrom oder dem wasserstoffhaltigen Einsatzgasstrom ein Dampfstrom zugeführt, insbesondere wenn der Reformierungsschritt ein autothermes Reformieren (ATR) umfasst.

Zusätzlicher Dampf wird vorzugsweise zugeführt, wenn der Reformierungsschritt ein autothermes Reformieren umfasst. Dadurch werden in vorteilhafter Weise Rußablagerungen auf dem Katalysator verhindert, welcher für die endotherme Dampfreformierung im ATR-Reaktor genutzt wird. Unter "zusätzlichem Dampf" wird dabei Dampf verstanden, welcher nicht durch die partielle Oxidationsreaktion reaktionsintern erzeugt wird. Entsprechend resultiert bei Zugabe eines Dampfstroms, abhängig davon ob die Zuführung des Dampfstroms vor oder nach der Zuführung des elektrolytisch erzeugten Wasserstoffstroms zum kohlenwasserstoffhaltigen Einsatzgasstroms erfolgt, ein dampfhaltiger und wasserstoffhaltiger Einsatzgasstrom. Letzterer kann, da er kohlenwasserstoffhaltig ist, auch als dampfhaltiger, wasserstoffhaltiger und kohlenwasserstoffhaltiger Einsatzgasstrom bezeichnet werden.

Eine bevorzugte Ausführungsform des erfindungsgemäßen Verfahrens ist dadurch gekennzeichnet, dass es sich bei dem kohlenwasserstoffhaltigen Einsatzgasstrom um einen

- Erdgasstrom oder einen
- Biogasstrom

handelt.

Insbesondere enthält der Erdgasstrom oder der Biogasstrom Methan ($CH_4$) als Hauptkomponente. Der Erdgasstrom oder Biogasstrom weist vorzugsweise mindestens 50 Vol.-% Methan auf, oder mindestens 75 Vol.-% Methan, oder mindestens 90 Vol.-% Methan, oder mindestens 95 Vol.-% Methan. Vorzugsweise handelt es sich bei dem Biogasstrom um einen vorbehandelten Biogasstrom, wobei die Vorbehandlung vorzugsweise darin besteht, die Konzentration an Methan zu erhöhen und die Konzentration von nicht erwünschten Begleitstoffen, insbesondere von Kohlendioxid, zu verringern.

Die vorgenannten Aufgaben werden ferner zumindest teilweise gelöst durch ein Verfahren zum Herstellen von Methanol, umfassend das Verfahren zum Herstellen von Synthesegas nach einer der vorgenannten Ausführungsformen, weiter umfassend den Schritt des Umsetzens des Synthesegasstroms an einem festen Methanolsynthese-Katalysator zu Rohmethanol, wobei das Rohmethanol zumindest Methanol ($CH_3OH$) und Wasser umfasst.

Vorzugsweise wird der hergestellte Synthesegasstrom unmittelbar, insbesondere ohne weiteren Modifikationsschritt oder ohne weitere Zuführung eines Wasserstoffstroms an einem festen Methanolsynthese-Reaktor zu Rohmethanol umgesetzt. Vorzugsweise weist das für die Methanolsynthese eingesetzte Synthesegas des Synthesegasstroms eine Stöchiometriezahl SN von 1,9 bis 2,5 auf, vorzugsweise von 2,0 bis 2,4 auf.

Das für die Methanolsynthese eingesetzte und erfindungsgemäß hergestellte Synthesegas des Synthesegasstroms kann auch als Synthesefrischgas bezeichnet werden. Dieses Synthesegas ist bezüglich seiner Zusammensetzung in den meisten Fällen von demjenigen Synthesegas zu unterscheiden, welches tatsächlich in den jeweiligen Methanolsynthese-Reaktor eingeschleust wird. Wie dem Fachmann bekannt, ist die Methanolsynthese im großtechnischen Maßstab in der Regel als Syntheseschleife ausgelegt, das heißt im Methanolsynthese-Reaktor nicht umgesetztes Synthesegas wird nach Abtrennung vom auskondensierten Rohmethanol zum Reaktoreingang zurückgeführt. Dieses zurückgeführte Synthesegas, auch als Recyclegas oder Rückführgas bezeichnet, wird mit dem Synthesefrischgas gemischt. Das resultierende gemischte Synthesegas, wie am Reaktoreingang zusammengesetzt, kann eine Stöchiometriezahl aufweisen, welche von dem oben genannten Intervall für das Synthesefrischgas abweicht.

Eine bevorzugte Ausführungsform des erfindungsgemäßen Verfahrens zum Herstellen von Methanol ist dadurch gekennzeichnet, dass das Rohmethanol in einem thermischen Trennverfahren in Reinmethanol und Wasser aufgetrennt wird.

Bei dem thermischen Trennverfahren handelt es sich um ein dem Fachmann bekanntes Verfahren wie Destillation oder Rektifikation.

Eine bevorzugte Ausführungsform des erfindungsgemäßen Verfahrens zum Herstellen von Methanol ist dadurch gekennzeichnet, dass das im thermischen Trennverfahren abgetrennte Wasser als Ausgangsmaterial für den elektrolytisch erzeugten Wasserstoff verwendet wird.

Vorzugsweise wird das im thermischen Trennverfahren abgetrennte Wasser aufgearbeitet, und anschließend als Ausgangsmaterial für den elektrolytisch erzeugten Wasserstoff verwendet. Häufig enthält das im thermischen Trennverfahren abgetrennte Wasser Natriumhydroxid (NaOH), welches beispielsweise für die Nutzung des Wassers in einer PEM-Elektrolyse entfernt werden muss. Wird der elektrolytisch erzeugte Wasserstoffstrom durch eine alkalische Elektrolyse erzeugt, ist die Entfernung von Natriumhydroxid nicht zwingend erforderlich, da für die alkalische Elektrolyse

hochkonzentrierte Kalilauge ($KOH_{aq}$) oder Natronlauge ($NaOH_{aq}$) als Elektrolysemedium verwendet wird.

Durch die Nutzung des im thermischen Trennverfahren abgetrennten Wassers als Ausgangsmaterial für den elektrolytisch erzeugten Wasserstoff wird die Prozessintegration verbessert, da weniger Ressourcen für die Bereitstellung von Wasser für die Wasserelektrolyse erforderlich sind.

Ferner kann das im thermischen Trennverfahren abgetrennte Wasser als Ausgangsmaterial für elektrolytisch erzeugten Sauerstoff verwendet werden.

Eine bevorzugte Ausführungsform des erfindungsgemäßen Verfahrens zum Herstellen von Methanol ist dadurch gekennzeichnet, dass beim Umsetzen des Synthesegasstroms an dem festen Methanolsynthese-Katalysator zu Rohmethanol ein Restgasstrom erzeugt wird, welcher nicht zu Rohmethanol umgesetztes Synthesegas enthält, und wobei von dem Restgasstrom ein Teil als Spülgasstrom abgetrennt wird, und wobei der Spülgasstrom einer Wasserstoffrückgewinnungsvorrichtung zugeführt wird, wodurch ein nicht elektrolytisch erzeugter Wasserstoffstrom erzeugt wird, und

- der nicht elektrolytisch erzeugte Wasserstoffstrom zumindest teilweise zusätzlich zu dem elektrolytisch erzeugten Wasserstoffstrom dem kohlenwasserstoffhaltigen Einsatzgasstrom zugeführt wird, wodurch der wasserstoffhaltige Einsatzgasstrom gemäß Schritt (c) erhalten wird, und/oder
- der nicht elektrolytisch erzeugte Wasserstoffstrom zumindest teilweise dem gemäß Schritt (d) erhaltenen Synthesegasstrom zugeführt wird.

Die Synthese von Methanol erfolgt im industriellen Maßstab wie vorgehend beschreiben üblicherweise innerhalb einer sogenannten Syntheseschleife. Die Umsetzung des Synthesegases am Methanolsynthese-Katalysator ist aufgrund der Einstellung eines thermodynamischen Gleichgewichts nicht vollständig, und am Ausgang des jeweiligen Reaktors wird neben kondensierbarem Rohmethanol auch ein nicht kondensierbarer Restgasstrom erhalten, welcher nicht umgesetztes Synthesegas enthält. Von diesem Restgasstrom wird ein Teil zum Reaktoreingang zur erneuten Umsetzung zu Rohmethanol zurückgeführt. Dieser zurückgeführte Strom wird als Rückführgasstrom oder Recyclegasstrom bezeichnet. Ein Teil wird als Spülgasstrom abgetrennt, um die Akkumulation von unter den Bedingungen der Methanolsynthese inerten Verbindungen in der Syntheseschleife zu vermeiden.

In vorteilhafter Weise wird dieser Spülgasstrom, welcher Wasserstoff aus dem nicht umgesetzten Synthesegas (Restgasstrom) enthält, einer Wasserstoffrückgewinnungsvorrichtung zugeführt, um damit einen nicht elektrolytisch erzeugten Wasserstoffstrom zu erzeugen.

Dieser wird gemäß einer Ausführungsform neben dem elektrolytisch erzeugten Wasserstoffstrom dem kohlenwasserstoffhaltigen Einsatzgasstrom zugeführt, wodurch der wasserstoffhaltige Einsatzgasstrom erhalten wird.

Alternativ oder zusätzlich wird der nicht elektrolytisch erzeugte Wasserstoffstrom dem Synthesegasstrom stromabwärts des jeweiligen Reformers (POX oder ATR) und stromaufwärts zur Methanolsynthese zugeführt.

Da insbesondere in einer Methanolanlage mit Syntheseschleife ein Spülgasstrom anfällt, kann dieser in vorteilhafter Weise prozessintern genutzt werden, und der für die Erzeugung des elektrolytisch erzeugten Wasserstoffstroms erforderliche Elektrolyseur kann entsprechend kleiner ausgelegt werden.

Bei der Wasserstoffrückgewinnungsvorrichtung kann es sich beispielsweise um eine Membraneinheit oder um eine Druckwechseladsorptionsvorrichtung (PSA) handeln. Vorzugsweise handelt es sich um eine PSA.

Eine bevorzugte Ausführungsform des erfindungsgemäßen Verfahrens zum Herstellen von Methanol ist in diesem Zusammenhang dadurch gekennzeichnet, dass eine gemäß Schritt (c) zum kohlenwasserstoffhaltigen Einsatzgasstrom zugeführte Menge an elektrolytisch erzeugtem Wasserstoffstrom und an nicht elektrolytisch erzeugtem Wasserstoffstrom so eingestellt wird, dass gemäß Schritt (d) ein Synthesegasstrom erhalten wird, welcher eine Stöchiometriezahl SN von 1,9 bis 2,5 aufweist, vorzugsweise von 2,0 bis 2,4 aufweist, wobei gilt

$$SN = \frac{n(H_2) - n(CO_2)}{n(CO) + n(CO_2)}, mit\ n\ in\ [mol].$$

Eine zur vorigen Ausführungsform alternative Ausführungsform des erfindungsgemäßen Verfahrens zum Herstellen von Methanol ist in diesem Zusammenhang dadurch gekennzeichnet, dass eine gemäß Schritt (c) zum kohlenwasserstoffhaltigen Einsatzgasstrom zugeführte Menge an elektrolytisch erzeugtem Wasserstoffstrom und eine zum Synthesegasstrom zugeführte Menge an nicht elektrolytisch erzeugtem Wasserstoffstrom so eingestellt wird, dass ein Synthesegasstrom erhalten wird, welcher eine Stöchiometriezahl SN von 1,9 bis 2,5 aufweist, vorzugsweise von 2,0 bis 2,4 aufweist, wobei gilt

$$SN = \frac{n(H_2) - n(CO_2)}{n(CO) + n(CO_2)}, mit\ n\ in\ [mol].$$

Die vorgenannten Aufgaben werden ferner zumindest teilweise gelöst durch eine Anlage zur Herstellung von Synthesegas, wobei die Anlage folgende in Wirkverbindung stehende Komponenten aufweist:

(a) Mittel konfiguriert zum Bereitstellen eines kohlenwasserstoffhaltigen Einsatzgasstroms;
(b) Einen Elektrolyseur konfiguriert zum Bereitstellen eines elektrolytisch erzeugten Wasserstoffstroms;
(c) Mittel konfiguriert zum Zuführen zumindest einen Teils des elektrolytisch erzeugten Wasserstoffstroms zu dem kohlenwasserstoffhaltigen Einsatzgasstrom, wodurch ein wasserstoffhaltiger Einsatzgasstrom erzeugbar ist;
(d) Einen Reaktor konfiguriert zum Umsetzen des wasserstoffhaltigen Einsatzgasstrom unter Beteiligung von Sauerstoff als Oxidationsmittel in einem Reformierungsschritt, wodurch ein Synthesegasstrom erzeugbar ist.

Die Anlage zur Herstellung von Synthesegas kann alternativ auch als Synthesegasproduktionsanlage bezeichnet werden.

Eine bevorzugte Ausführungsform der Anlage ist dadurch gekennzeichnet, dass der Reaktor (d) ein autothermer Reformer ist oder ein für eine partielle Oxidation konfigurierter Reaktor ist.

Eine bevorzugte Ausführungsform der Anlage ist dadurch gekennzeichnet, dass die Mittel (c) so konfiguriert sind, dass eine zum kohlenwasserstoffhaltigen Einsatzgasstrom zuführbare Menge an elektrolytisch erzeugtem Wasserstoffstrom so einstellbar ist, dass gemäß (d) ein Synthesegasstrom erzeugbar ist, welcher eine Stöchiometriezahl SN von 1,9 bis 2,5 aufweist, vorzugsweise von 2,0 bis 2,4 aufweist, wobei gilt

$$SN = \frac{n(H_2) - n(CO_2)}{n(CO) + n(CO_2)}, mit\ n\ in\ [mol].$$

Eine bevorzugte Ausführungsform der Anlage ist dadurch gekennzeichnet, dass die Anlage keine Mittel zum Entfernen von als Verunreinigung auftretendem Sauerstoff aus dem elektrolytisch erzeugbaren Wasserstoffstrom beinhaltet.

Eine bevorzugte Ausführungsform der Anlage ist dadurch gekennzeichnet, dass der Elektrolyseur zum Bereitstellen eines elektrolytisch erzeugten Sauerstoffstroms konfiguriert ist, und die Anlage Mittel zum Verwenden des Sauerstoffstroms als Oxidationsmittel im Reaktor (d) zum Umsetzen des wasserstoffhaltigen Einsatzgasstrom zum Synthesegasstrom umfasst.

Eine bevorzugte Ausführungsform der Anlage ist dadurch gekennzeichnet, dass die Anlage eine Vorrichtung zur Luftzerlegung umfasst, wobei durch die Vorrichtung zur Luftzerlegung ein Sauerstoffstrom erzeugbar ist, und die Anlage Mittel zum Verwenden des Sauerstoffstroms als Oxidationsmittel im Reaktor zum Umsetzen des wasserstoffhaltigen Einsatzgasstrom zum Synthesegasstrom umfasst.

Alternativ kann der Sauerstoffstrom durch eine pipeline zur Verfügung gestellt werden.

Die vorgenannten Aufgaben werden ferner zumindest teilweise gelöst durch eine Methanolanlage zur Herstellung von Methanol, umfassend eine Anlage zur Herstellung von Synthesegas nach einer der vorgenannten Ausführungsformen, dadurch gekennzeichnet, dass die Methanolanlage einen Methanolsynthese-Reaktor aufweist, wobei der Methanolsynthese-Reaktor zum Umsetzen des durch die Anlage erzeugbaren Synthesegases zu Rohmethanol konfiguriert ist, wobei das Rohmethanol zumindest Methanol ($CH_3OH$) und Wasser enthält.

Eine bevorzugte Ausführungsform der Methanolanlage ist dadurch gekennzeichnet, dass die Methanolanlage eine thermische Trennvorrichtung aufweist, wobei die thermische Trennvorrichtung zum Auftrennen des Rohmethanols in Reinmethanol und Wasser konfiguriert ist.

Eine bevorzugte Ausführungsform der Methanolanlage ist dadurch gekennzeichnet, dass die Methanolanlage Mittel zum Verwenden des abgetrennten Wassers als Ausgangsmaterial für den elektrolytisch erzeugbaren Wasserstoff umfasst.

Eine bevorzugte Ausführungsform der Methanolanlage ist dadurch gekennzeichnet, dass durch das Umsetzen des durch die Anlage erzeugbaren Synthesegases zu Rohmethanol ein Restgasstrom erzeugbar ist, wobei der Restgasstrom nicht zu Rohmethanol umgesetztes Synthesegas enthält, und die Methanolanlage Mittel zum Abtrennen eines Spülgasstroms von dem Restgasstrom aufweist, und die Methanolanlage eine Wasserstoffrückgewinnungsvorrichtung aufweist, und Mittel zum Zuführen des Spülgasstroms zu der Wasserstoffrückgewinnungsvorrichtung aufweist, wodurch ein nicht elektrolytisch erzeugter Wasserstoffstrom mittels der Wasserstoffrückgewinnungsvorrichtung aus dem Spülgasstrom erzeugbar ist, und die Methanolanlage

- Mittel zum Zuführen zumindest eines Teils des nicht elektrolytisch erzeugten Wasserstoffstroms zusätzlich zu dem elektrolytisch erzeugten Wasserstoffstrom zu dem kohlenwasserstoffhaltigen Einsatzgasstrom aufweist, wodurch der wasserstoffhaltige Einsatzgasstrom gemäß (c) erzeugbar ist und/oder
- Mittel zum Zuführen zumindest eines Teils des nicht elektrolytisch erzeugten Wasserstoffstroms zu dem gemäß (d) erzeugbaren Synthesegasstrom aufweist.

Eine bevorzugte Ausführungsform der Methanolanlage ist in diesem Zusammenhang dadurch gekennzeichnet, dass die Mittel (c) und die Mittel zum Zuführen des nicht elektrolytisch erzeugten Wasserstoffstroms so konfiguriert sind, dass eine zum kohlenwasserstoffhaltigen Einsatzgasstrom zuführbare Menge an elektrolytisch erzeugtem Wasserstoffstrom und an nicht elektrolytisch erzeugtem Wasserstoffstrom so einstellbar ist, dass gemäß (d) ein Synthesegasstrom erzeugbar ist, welcher eine Stöchiometriezahl SN von 1,9 bis 2,5 aufweist, vorzugsweise von 2,0 bis 2,4 aufweist, wobei gilt

$$SN = \frac{n(H_2) - n(CO_2)}{n(CO) + n(CO_2)}, mit\ n\ in\ [mol].$$

Eine zu voriger Ausführungsform alternative Ausführungsform der Methanolanlage ist in diesem Zusammenhang dadurch gekennzeichnet, dass die Mittel (c) und die Mittel zum Zuführen zumindest eines Teils des nicht elektrolytisch erzeugten Wasserstoffstroms zu dem gemäß (d) erzeugbaren Synthesegasstrom so konfiguriert sind, dass ein Synthesegasstrom erzeugbar ist, welcher eine Stöchiometriezahl SN von 1,9 bis 2,5 aufweist, vorzugsweise von 2,0 bis 2,4 aufweist, wobei gilt

$$SN = \frac{n(H_2) - n(CO_2)}{n(CO) + n(CO_2)}, mit\ n\ in\ [mol].$$

**Ausführungsbeispiele**

Die Erfindung wird im Folgenden durch Ausführungsbeispiele und Zahlenbeispiele näher erläutert, ohne den Gegenstand der Erfindung dadurch zu beschränken. Weitere Merkmale, Vorteile und Anwendungsmöglichkeiten der Erfindung ergeben sich aus der nachfolgenden Beschreibung der Ausführungsbeispiele in Zusammenhang mit den Zeichnungen und der Zahlenbeispiele. In den Figuren sind funktionell und/oder strukturell gleiche oder zumindest ähnliche Bestandteile mit gleichen Bezugszeichen versehen.

Es zeigt

Figur 1 ein Blockfließbild eines Verfahrens zur Synthesegasherstellung und Methanolherstellung nach dem Stand der Technik unter Verwendung von PSA-Wasserstoff,

Figur 2 ein Blockfließbild eines Verfahrens zur Synthesegasherstellung und Methanolherstellung nach dem Stand der Technik unter Verwendung von elektrolytisch erzeugtem Wasserstoff,

Figur 3 ein Blockfließbild eines Verfahrens zur Synthesegasherstellung und Methanolherstellung gemäß der Erfindung, und

Figur 4 ein Blockfließbild eines weiteren Verfahrens zur Synthesegasherstellung und Methanolherstellung gemäß der Erfindung.

Figur 1 zeigt ein stark vereinfachtes Blockfließbild eines Beispiels eines Verfahrens 1 zur Synthesegasherstellung und nachfolgenden Methanolherstellung gemäß konventionellen Verfahren aus dem Stand der Technik.

Über Leitung 20 wird ein kohlenwasserstoffhaltiger Einsatzgasstrom, hier ein Erdgasstrom, herangeführt und zunächst in Kompressor 10 verdichtet. Das verdichtete Einsatzgas wird anschließend über Leitung 21 weitergeführt und in einer Einheit zur Aufreinigung von Erdgas 11 von unerwünschten Begleitstoffen befreit. Aufreinigungseinheit 11 umfasst beispielsweise eine Hydrodesulfurierungsanlage zur Entfernung von Schwefelverbindungen. Das gereinigte Einsatzgas wird über Leitung 22 weitergeführt und mit über Leitung 31 zugeführtem Dampf gemischt. Das Gemisch aus Einsatzgas und Dampf wird anschließend in einem autothermen Reformer 12 zu einem Synthesegasstrom umgesetzt. Der autotherme Reformer wird über eine nicht gezeigte Leitung mit Luft, sauerstoffangereicherter Luft oder reinem Sauerstoff versorgt. Das im autothermen Reformer erzeugte Synthesegas wird über Leitung 23 weitergeführt und in

einer Einheit zur Wärmerückgewinnung 13 abgekühlt. In der Einheit zur Wärmerückgewinnung 13 wird gleichzeitig Prozesswasser aus Leitung 30 zu Dampf umgewandelt, welcher über Leitung 31 weitergeführt und wie erwähnt mit dem kohlenwasserstoffhaltigen Einsatzgasstrom in Leitung 22 zusammengeführt wird.

Der abgekühlte, die Einheit zur Wärmerückgewinnung 13 verlassende Synthesegasstrom weist eine Stöchiometriezahl von deutlich unter 2,0 auf und kann daher nicht unmittelbar für eine nachfolgende Methanolsynthese verwendet werden. Aus diesem Grund wird von dem Synthesegasstrom der Leitung 24 ein Teilstrom über Leitung 25 abgenommen und einer Einheit zur Druckwechseladsorption 14 zugeführt. In der Einheit zur Druckwechseladsorption 14 wird ein Strom von weitgehend reinem Wasserstoff erzeugt, welcher über Leitung 26 aus der Druckwechseladsorptionseinheit 14 ausgeleitet wird. Gleichzeitig wird in der Einheit zur Druckwechseladsorption 14 ein Abgasstrom erzeugt (nicht gezeigt), welcher beispielsweise partiell im autothermen Reformer 12 verwendet werden kann oder aber zur Unterfeuerung in einem separaten Fired Heater eingesetzt werden kann.

Der über Leitung 26 aus der Einheit zur Druckwechseladsorption 14 ausgeleitete Wasserstoffstrom wird mit dem Synthesegasstrom aus Leitung 24 zusammengeführt, wodurch ein mit Wasserstoff angereicherter Synthesegasstrom resultiert, welcher über Leitung 27 weitergeführt wird. Der mit Wasserstoff angereicherte Synthesegasstrom in Leitung 27 weist eine Stöchiometriezahl von über 2,0 auf und kann daher für eine nachfolgende Methanolsynthese verwendet werden. Entsprechend wird der Synthesegasstrom über Leitung 27 in eine Einheit zur Methanolsynthese 15 eingeleitet. Die Einheit zur Methanolsynthese 15 kann einen oder mehrere hintereinander geschaltete Methanolsynthese-Reaktoren aufweisen. In dem oder den Methanolsynthese-Reaktor(en) werden der Wasserstoff und die Kohlenstoffoxide aus dem mit Wasserstoff angereicherten Synthesegasstrom an einem festen Methanolsynthese-Katalysator zu Rohmethanol, einem Gemisch aus Methanol und Wasser, umgesetzt. Das Rohmethanol wird anschließend über Leitung 28 weitergeführt und einer Destillationseinheit 16 zugeführt. Destillationseinheit 16 umfasst beispielsweise eine Rektifikationskolonne. In der Destillationseinheit wird das Methanol von Wasser und unerwünschten Nebenprodukten getrennt. Ein Strom von reinem Methanol wird über Leitung 29 aus der Destillationseinheit 29 ausgeleitet, neben einem Strom von Prozesswasser, welcher über Leitung 30 ausgeleitet wird. Das Prozesswasser in Leitung 30 wird in der Einheit zur Wärmerückgewinnung 13 in Dampf umgewandelt.

Figur 2 zeigt ein stark vereinfachtes Blockfließbild eines Beispiels eines Verfahrens 2 zur Synthesegasherstellung und nachfolgenden Methanolherstellung gemäß dem Prinzip wie in EP 3 658 494 B1 beschrieben.

Im Vergleich zu Verfahren 1 wird der zur Einstellung der Stöchiometriezahl des durch Reformierung hergestellten Synthesegases benötigte Wasserstoff durch einen Elektrolyseur 18 erzeugt. Entsprechend ist es nicht erforderlich, von dem abgekühlten Synthesegasstrom in Leitung 24 einen Teilstrom abzuzweigen und daraus Wasserstoff in einer Einheit zur Druckwechseladsorption, wie für Figur 1 beschrieben, zu erzeugen.

Dementsprechend wird über Leitung 32 ein Rohwasserstrom herangeführt, welcher in einer Einheit zur Wasseraufbereitung 17 gereinigt wird, beispielsweise entsalzt wird. Der daraus resultierende Reinwasserstrom wird über Leitung 33 weitergeführt und im Elektrolyseur 18 einer Wasserelektrolyse unterzogen, wodurch ein elektrolytisch erzeugter Wasserstoffstrom und ein elektrolytisch erzeugter Sauerstoffstrom erzeugt werden. Bei der Elektrolyse kann es sich beispielsweise um eine PEM-Elektrolyse oder eine alkalische Elektrolyse handeln. Aus dem Elektrolyseur 18 wird über Leitung 35 ein Sauerstoffstrom abgezogen, welcher als Oxidationsmittel im autothermen Reformer 12 verwendet und daher diesem zugeführt wird. Der ebenfalls im Elektrolyseur elektrolytisch erzeugte Wasserstoffstrom wird über Leitung 34 weitergeführt. Der Wasserstoffstrom in Leitung 34 enthält Sauerstoff als Verunreinigung, beispielsweise 1 Vol.-%, und gegebenenfalls Restmengen an nicht im Gas-Flüssigkeit-Abscheider des Elektrolyseurs 18 entferntem Wasser. Der Wasserstoffstrom wird daher einer Einheit zur Aufreinigung von Elektrolyse-Wasserstoff 19 zugeführt. In dieser Aufreinigungseinheit 19 wird der als Verunreinigung anwesende Sauerstoff zunächst katalytisch mit Wasserstoff des Wasserstoffstroms zu Wasser umgesetzt. Anschließend wird dieses katalytisch erzeugte Wasser und vorgenanntes in Restmengen enthaltene Wasser adsorptiv gebunden, beispielsweise an einem Molekularsieb. Der die Aufreinigungseinheit 19 verlassende elektrolytisch erzeugte Wasserstoffstrom ist entsprechend frei von Sauerstoff und trocken, also wasserfrei. Dieser gereinigte Wasserstoffstrom wird über Leitung 36 weitergeführt und mit dem abgekühlten Synthesegasstrom aus Leitung 24 zusammengeführt. Es resultiert ein mit Wasserstoff angereicherter Synthesegasstrom, welcher eine Stöchiometriezahl von größer als 2,0 aufweist und daher für die nachfolgende Methanolsynthese in der Einheit zur Methanolsynthese 15 verwendet werden kann.

Figur 3 zeigt ein stark vereinfachtes Blockfließbild eines Beispiels eines Verfahrens 3 zur Synthesegasherstellung und nachfolgenden Methanolherstellung gemäß der Erfindung.

Die Verfahrensführung gemäß dem Verfahren 3 unterscheidet sich von dem Verfahren 2 insbesondere darin, dass der von dem Elektrolyseur 18 elektrolytisch erzeugte Wasserstoffstrom über Leitung 38 dem aufgereinigten kohlenwasserstoffhaltigen Einsatzgasstrom in Leitung 22 zugeführt wird. Wie für Figur 1 im Detail beschrieben, wird auch gemäß Verfahren 3 ein kohlenwasserstoffhaltiger Einsatzgasstrom, hier ein Erdgasstrom, über Leitung 20 herangeführt. Der kohlenwasserstoffhaltige Einsatzgasstrom wird anschließend über Kompressor 10 verdichtet, über Leitung 21 weitergeführt und in der Einheit zur Aufreinigung des Einsatzgases 11 aufgereinigt. Die Aufreinigungseinheit 11 beinhaltet eine Hydrodesulfurierungsanlage zur Entfernung von Schwefelverbindungen aus dem Einsatzgasstrom. Der aufgereinigte, kohlenwasserstoffhaltige Einsatzgasstrom wird über Leitung 22 weitergeführt und wie oben beschrieben mit dem elektrolytisch erzeugten Wasserstoffstrom aus Leitung 38 zusammengeführt. Dem so resultierenden, wasserstoffhaltigen und kohlenwasserstoffhaltigen Einsatzgasstrom (wasserstoffhaltiger Einsatzgasstrom im Sinne der Erfindung) wird ferner Dampf aus Leitung 39 zugemischt. Es resultiert in Leitung 22 ein dampfhaltiger, kohlenwasserstoffhaltiger und wasserstoffhaltiger Einsatzgasstrom. Dieser Strom wird im autothermen Reformer zu Synthesegas umgesetzt. Dem autothermen Reformer wird auch elektrolytisch erzeugter Sauerstoff über Leitung 35 als Oxidationsmittel zugeführt. Dem autothermen Reformer wird darüber hinaus über eine nicht gezeigte Leitung der für die Reaktion noch fehlende Sauerstoff, in Form von Luft, sauerstoffangereicherter Luft oder reinem Sauerstoff, zugeführt.

Da Wasserstoff im autothermen Reformer nicht reagiert oder nur in geringem Maße mit Sauerstoff zu Wasser reagiert, weist das Synthesegas am Ausgang des autothermen Reformers eine hohe Stöchiometriezahl SN von mehr als 1,9 auf. Da für den autothermen Reformer in jedem Fall Sauerstoff als Oxidationsmittel benötigt wird, ist es nicht erforderlich den elektrolytisch erzeugten Wasserstoffstrom in Leitung 38 aufzureinigen, so wie für das Verfahren 2 beschrieben. Da dem wasserstoffhaltigen Einsatzgasstrom ein Dampfstrom zugeführt wird beziehungsweise im Zuge der partiellen Oxidation im autothermen Reformer 12 Dampf erzeugt (und umgesetzt) wird, ist es auch nicht erforderlich den elektrolytisch erzeugten Wasserstoffstrom zu trocknen. Entsprechend ist für das erfindungsgemäße Verfahren 3 keine Aufreinigungseinheit 19 wie für Figur 2 beschrieben erforderlich.

Der Synthesegasstrom, welcher eine Stöchiometriezahl von mehr als 1,9 aufweist, wird über Leitung 23 weitergeführt und in der Einheit zur Wärmerückgewinnung 13 abgekühlt. Der abgekühlte Synthesegasstrom wird über Leitung 40 weitergeführt und kann anschließend direkt in der Einheit zur Methanolsynthese 15 zur Herstellung von Methanol verwendet werden.

Das in Fig. 3 gezeigte Verfahren stellt ferner eine Optimierung und Alternative zur Verwendung des in der Destillationseinheit 16 anfallenden Prozesswassers da. Das Prozesswasser wird gemäß dem in Fig. 3 gezeigten Verfahren einer Wasseraufbereitungseinheit 17 zugeführt. In Abhängigkeit von dessen Qualität kann es auch direkt als feed für den Elektrolyseur verwendet werden, unter Umgehung der Wasseraufbereitungseinheit 17. In diesem Fall wird das Prozesswasser über Leitung 37 direkt der Leitung 33 zugeführt (nicht gezeigt). In jedem Fall wird die erforderliche Menge an Rohwasser (Leitung 32) und/oder Reinwasser (Leitung 33) verringert. Je nach verfügbarer Menge an Prozesswasser aus Destillationseinheit 16 kann dieses zusätzlich zur Erzeugung von Dampf in der Wärmerückgewinnungseinheit 13 verwendet werden (in Figur 3 nicht gezeigt).

Figur 4 zeigt ein stark vereinfachtes Blockfließbild eines Beispiels eines weiteren Verfahrens, hier Verfahren 4, zur Synthesegasherstellung und nachfolgenden Methanolherstellung gemäß der Erfindung.

Die Verfahrensführung gemäß dem in Figur 4 gezeigten Verfahren 4 unterscheidet sich von dem Verfahren 3 insbesondere darin, dass aus der Methanolsynthese 15 ein Spülgasstrom über Leitung 41 abgezogen und einer Vorrichtung für Druckwechseladsorption (PSA) 9 zugeführt wird. Der Spülgasstrom wird von einem Restgasstrom (nicht gezeigt) abgetrennt, welcher im Wesentlichen nicht in der Methanolsynthese 9 umgesetztes Synthesegas enthält. Die Vorrichtung zur Druckwechseladsorption 9 erzeugt einen Wasserstoffstrom und einen Abgasstrom (Abgasstrom nicht gezeigt). Der Wasserstoffstrom, im Sinne der Erfindung ein nicht elektrolytisch erzeugter Wasserstoffstrom, wird über Leitung 42 weitergeführt und in Leitung 40 mit dem im autothermen Reformer 12 erzeugten Synthesegasstrom zusammengeführt.

Alternativ oder zusätzlich könnte der aus der PSA 9 abgezogene Wasserstoffstrom auch dem kohlenwasserstoffhaltigen Einsatzgasstrom in Leitung 22, zusätzlich zu dem elektrolytisch erzeugten Wasserstoffstrom, zugeführt werden (siehe gestrichelt dargestellte Leitung 42').

Durch die Nutzung des Spülgases zur Erzeugung von Wasserstoff durch Druckwechseladsorption kann der Elektrolyseur entsprechend kleiner ausgelegt werden, da dieser entsprechend weniger Wasserstoff zum Anpassen der Stöchiometriezahl des Synthesegasstroms produzieren muss.

Die folgenden Zahlenbeispiele basieren auf Simulationsdaten und dienen der weiteren Erläuterung der Erfindung. Die Simulationsdaten wurden mit Hilfe der Software AspenPlus® generiert.

Die folgende Tabelle zeigt Simulationsdaten für zwei Vergleichsbeispiele P1 und P2 sowie zwei erfindungsgemäße Beispiele P3 und P4, jeweils für eine Verfahrensführung mit POX-Reaktor. P1 entspricht prinzipiell der Verfahrensführung gemäß Figur 1 mit Erzeugung eines Wasserstoffstroms durch Druckwechseladsorption von reformiertem Synthesegas. P2 entspricht prinzipiell der Verfahrensführung gemäß Figur 2 mit Erzeugung eines Wasserstoffstroms durch Elektrolyse und Zuführung des Wasserstoffstroms stromabwärts des POX-Reaktors. P3 und P4 entsprechen prinzipiell der Verfahrensführung gemäß Figur 3 (Erfindung) mit Erzeugung eines Wasserstoffstroms durch Elektrolyse und Zuführung des Wasserstoffstroms stromaufwärts des POX-Reaktors.

Ströme sind häufig als Stoffmengenströme (mole flow) in kilomol pro Stunde (kmol/h) angegeben.

| | P1 (Vergleich) | P2 (Vergleich) | P3 (Erfindung) | P4 (Erfindung) |
|---|---|---|---|---|
| Erdgasstrom (kmol/h) | 404,0 | 316,0 | 316,0 | 316,0 |

(fortgesetzt)

| | P1 (Vergleich) | P2 (Vergleich) | P3 (Erfindung) | P4 (Erfindung) |
|---|---|---|---|---|
| Wasserstoffstrom Elektrolyseur (kmol/h) | n/a | 120,0 | 120,0 | 150,0 |
| Sau erstoffstrom (t/h) | 300,0 | 239,0 | 249,9 | 252,7 |
| Synthesegasstrom zu PSA | 21,5% | n/a | n/a | n/a |
| Synthesegasstrom (kmol/h) | 1031,3 | 1032,2 | 1007,7 | 1031,8 |
| Stöchiometriezahl SN | 1,99 | 1,97 | 1,91 | 1,98 |

Der erforderliche Erdgasstrom ist für P1 deutlich höher, da ein relativ großer Anteil des erzeugten Synthesegasstroms (21,5 %) für die Erzeugung des Wasserstoffstroms durch Druckwechseladsorption (PSA) abgezweigt werden muss.

Der Vergleich zwischen P2 und P3 zeigt, dass bei Zuführung des gleichen Wasserstoffstroms stromaufwärts des POX-Reaktors (P3) eine etwas geringere Synthesegasmenge produziert wird und das resultierende Synthesegas eine etwas geringere Stöchiometriezahl aufweist. Um diesen Effekt zu kompensieren, ist die Erhöhung der Wasserstoffstrom-Menge von 120 kmol/hr auf 150 kmol/hr (gemäß P4) erforderlich. Die dadurch bedingte Steigerung der Betriebskosten (OPEX) kann durch die entsprechende Verringerung der Investitionskosten (CAPEX) kompensiert werden, da erfindungsgemäß keine dem Elektrolyseur nachgeschaltete Aufreinigungseinheit zur Entfernung von Sauerstoff erforderlich ist. Eine solche Aufreinigungseinheit steigert auch die Betriebskosten (OPEX) der entsprechenden Anlage.

Die folgende Tabelle zeigt Simulationsdaten für zwei Vergleichsbeispiele A1 und A2 sowie ein erfindungsgemäßes Beispiel A3, jeweils für eine Verfahrensführung mit autothermem Reformer (ATR-Reaktor). A1 entspricht prinzipiell der Verfahrensführung gemäß Figur 1 mit Erzeugung eines Wasserstoffstroms durch Druckwechseladsorption (PSA) von reformiertem Synthesegas. Zusätzlich wird, nicht in Figur 1 gezeigt, ein weiterer Wasserstoffstrom durch eine PSA durch Nutzung eines bei der Methanolsynthese anfallenden Spülgasstroms erzeugt. Dieser nicht elektrolytisch erzeugte Wasserstoffstrom wird dem Synthesegasstrom stromabwärts des autothermen Reformers, stromaufwärts der Methanolsynthese, zugeführt.

A2 entspricht der Verfahrensführung gemäß Figur 2 mit Erzeugung eines Wasserstoffstroms durch Elektrolyse und Zuführen dieses Wasserstoffstroms stromabwärts des autothermen Reformers. Zusätzlich wird, nicht in Figur 2 gezeigt, ein weiterer Wasserstoffstrom durch eine PSA durch Nutzung des bei der Methanolsynthese anfallenden Spülgasstroms erzeugt. Dieser nicht elektrolytisch erzeugte Wasserstoffstrom wird dem Synthesegasstrom stromabwärts des autothermen Reformers, stromaufwärts der Methanolsynthese, zugeführt.

A3 entspricht der Verfahrensführung gemäß Figur 4 (Erfindung) mit Erzeugung eines Wasserstoffstroms durch Elektrolyse und Zuführen dieses Wasserstoffstroms stromaufwärts des autothermen Reformers. Zusätzlich wird ein weiterer Wasserstoffstrom durch eine PSA durch Nutzung des bei der Methanolsynthese anfallenden Spülgasstroms erzeugt. Dieser nicht elektrolytisch erzeugte Wasserstoffstrom wird dem Synthesegasstrom stromabwärts des autothermen Reformers, stromaufwärts der Methanolsynthese, zugeführt.

Die Beispiele A1 bis A3 zeigen auch die Synthese von Methanol aus dem erzeugten Synthesegas in einem Syntheseloop mit wassergekühltem Reaktor bei einer Rezirkulationsrate von 1,6. Die Rezirkulationsrate (recycle ratio, RR) stellt den Quotienten des Stroms von Recyclegas (zum Reaktoreingang zurückgeführtes, am Ausgang nicht umgesetztes Restgas) und frisch zugeführtem Synthesegas dar.

Ströme sind häufig als Stoffmengenströme (mole flow) in kilomol pro Stunde (kmol/h) angegeben.

| | A1 (Vergleich) | A2 (Vergleich) | A3 (Erfindung) |
|---|---|---|---|
| Erdgasstrom (kmol/h) | 7741 | 7394 | 7408 |
| Wasserstoffstrom aus Elektrolyseur (kmol/h) | n/a | 880 | 1000 |
| Wasserstoffstrom aus PSA (kmol/h) | 1650 | 650 | 700 |
| Sau erstoffstrom (t/h) | 137 | 127 | 128 |
| Synthesegasstrom zu PSA (kmol/h) | 1800 | n/a | n/a |
| Synthesegasstrom (kmol/h) | 22943 | 23001 | 23038 |
| Stöchiometriezahl SN | 2,4 | 2,4 | 2,5 |
| Methanolprodukt (t/d) | 5001 | 5004 | 5001 |

Der Vergleich zwischen A2 und A3 zeigt, dass bei Zuführung des Wasserstoffstroms zum Erdgasstrom stromaufwärts des autothermen Reformers eine größere Wasserstoffmenge zur Produktion der gleichen Methanol-Menge (bzw. SynthesegasMenge) erforderlich ist. Die dadurch bedingte Steigerung der Betriebskosten (OPEX) kann durch die entsprechende Verringerung der Investitionskosten (CAPEX) kompensiert werden, da erfindungsgemäß keine dem Elektrolyseur nachgeschaltete Aufreinigungseinheit zur Entfernung von Sauerstoff erforderlich ist. Eine solche Aufreinigungseinheit steigert auch die Betriebskosten (OPEX) der entsprechenden Anlage.

**Bezugszeichenliste**

| | |
|---|---|
| 1, 2, 3 | Verfahren |
| 9 | Druckwechseladsorption |
| 10 | Kompressor |
| 11 | Aufreinigung Einsatzgas |
| 12 | Autothermer Reformer |
| 13 | Wärmerückgewinnung |
| 14 | Druckwechseladsorption |
| 15 | Methanolsynthese |
| 16 | Destillation |
| 17 | Wasseraufbereitung |
| 18 | Elektrolyseur |
| 19 | Aufreinigung Elektrolyse-Wasserstoff |
| 20 bis 42, 42’ | Leitung |

**Patentansprüche**

**1.** Verfahren zum Herstellen von Synthesegas, insbesondere von Synthesegas für die Methanolsynthese, umfassend die Schritte:

(a) Bereitstellen eines kohlenwasserstoffhaltigen Einsatzgasstroms;
(b) Bereitstellen eines elektrolytisch erzeugten Wasserstoffstroms;
(c) Zuführen zumindest eines Teils des elektrolytisch erzeugten Wasserstoffstroms zu dem kohlenwasserstoffhaltigen Einsatzgasstrom, wodurch ein wasserstoffhaltiger Einsatzgasstrom erhalten wird;
(d) Umsetzen des wasserstoffhaltigen Einsatzgasstroms unter Beteiligung von Sauerstoff als Oxidationsmittel in einem Reformierungsschritt zu einem Synthesegasstrom.

**2.** Verfahren nach Anspruch 1, **dadurch gekennzeichnet, dass** der Reformierungsschritt ein autothermes Reformieren (ATR) oder eine partielle Oxidation (POX) des wasserstoffhaltigen Einsatzgasstroms umfasst.

**3.** Verfahren nach Anspruch 1 oder 2, **dadurch gekennzeichnet, dass** eine gemäß Schritt (c) zum kohlenwasserstoffhaltigen Einsatzgasstrom zugeführte Menge an elektrolytisch erzeugtem Wasserstoffstrom so eingestellt wird, dass gemäß Schritt (d) ein Synthesegasstrom erhalten wird, welcher eine Stöchiometriezahl SN von 1,9 bis 2,5 aufweist, vorzugsweise von 2,0 bis 2,4 aufweist, wobei gilt

$$SN = \frac{n(H_2) - n(CO_2)}{n(CO) + n(CO_2)}, mit\ n\ in\ [mol].$$

**4.** Verfahren nach einem der vorherigen Ansprüche, **dadurch gekennzeichnet, dass** der elektrolytisch erzeugte Wasserstoffstrom als Verunreinigung Sauerstoff enthält, und der als Verunreinigung enthaltene Sauerstoff vor dem Zuführen des elektrolytisch erzeugten Wasserstoffstroms zu dem kohlenwasserstoffhaltigen Einsatzgasstrom gemäß Schritt (c) nicht entfernt wird.

**5.** Verfahren nach einem der vorherigen Ansprüche, **dadurch gekennzeichnet, dass** der elektrolytisch erzeugte Wasserstoffstrom als Verunreinigung bis zu 5 Vol.-% Sauerstoff enthält, oder 0,01 bis 5 Vol.-% Sauerstoff enthält, oder 0,1 bis 3 Vol.-% Sauerstoff enthält, oder 0,1 bis 1 Vol.-% Sauerstoff enthält.

**6.** Verfahren nach einem der vorherigen Ansprüche, **dadurch gekennzeichnet, dass** das Verfahren das Bereitstellen eines elektrolytisch erzeugten Sauerstoffstroms umfasst, wobei der elektrolytisch erzeugte Sauerstoffstrom als Oxidationsmittel in Schritt (d) verwendet wird.

**7.** Verfahren nach einem der Ansprüche 1 bis 5, **dadurch gekennzeichnet, dass** das Verfahren das Bereitstellen eines durch Luftzerlegung erzeugten Sauerstoffstroms umfasst, wobei der durch Luftzerlegung erzeugte Sauerstoffstrom als Oxidationsmittel in Schritt (d) verwendet wird.

**8.** Verfahren nach einem der vorherigen Ansprüche, **dadurch gekennzeichnet, dass** dem kohlenwasserstoffhaltigen Einsatzgasstrom oder dem wasserstoffhaltigen Einsatzgasstrom ein Dampfstrom zugeführt wird.

**9.** Verfahren nach einem der vorherigen Ansprüche, **dadurch gekennzeichnet, dass** es sich bei dem kohlenwasserstoffhaltigen Einsatzgasstrom um einen

- Erdgasstrom oder einen
- Biogasstrom

handelt.

**10.** Verfahren (3, 4) zum Herstellen von Methanol, umfassend das Verfahren zum Herstellen von Synthesegas nach einem der vorherigen Ansprüche, weiter umfassend den Schritt des Umsetzens des Synthesegasstroms an einem festen Methanolsynthese-Katalysator zu Rohmethanol, wobei das Rohmethanol zumindest Methanol ($CH_3OH$) und Wasser umfasst.

**11.** Verfahren nach Anspruch 10, **dadurch gekennzeichnet, dass** das Rohmethanol in einem thermischen Trennverfahren in Reinmethanol und Wasser aufgetrennt wird.

**12.** Verfahren nach Anspruch 10 oder 11, **dadurch gekennzeichnet, dass** das im thermischen Trennverfahren abgetrennte Wasser als Ausgangsmaterial für den elektrolytisch erzeugten Wasserstoff verwendet wird.

**13.** Verfahren nach einem der Ansprüche 10 bis 12, **dadurch gekennzeichnet, dass** beim Umsetzen des Synthesegasstroms an dem festen Methanolsynthese-Katalysator zu Rohmethanol ein Restgasstrom erzeugt wird, welcher nicht zu Rohmethanol umgesetztes Synthesegas enthält, und wobei von dem Restgasstrom ein Teil als Spülgasstrom abgetrennt wird, und wobei der Spülgasstrom einer Wasserstoffrückgewinnungsvorrichtung zugeführt wird, wodurch ein nicht elektrolytisch erzeugter Wasserstoffstrom erzeugt wird, und

- der nicht elektrolytisch erzeugte Wasserstoffstrom zumindest teilweise zusätzlich zu dem elektrolytisch erzeugten Wasserstoffstrom dem kohlenwasserstoffhaltigen Einsatzgasstrom zugeführt wird, wodurch der wasserstoffhaltige Einsatzgasstrom gemäß Schritt (c) erhalten wird, und/oder
- der nicht elektrolytisch erzeugte Wasserstoffstrom zumindest teilweise dem gemäß Schritt (d) erhaltenen Synthesegasstrom zugeführt wird.

**14.** Anlage zur Herstellung von Synthesegas, wobei die Anlage folgende in Wirkverbindung stehende Komponenten aufweist:

(a) Mittel konfiguriert zum Bereitstellen eines kohlenwasserstoffhaltigen Einsatzgasstroms;
(b) Einen Elektrolyseur (18) konfiguriert zum Bereitstellen eines elektrolytisch erzeugten Wasserstoffstroms;
(c) Mittel konfiguriert zum Zuführen zumindest einen Teils des elektrolytisch erzeugten Wasserstoffstroms zu dem kohlenwasserstoffhaltigen Einsatzgasstrom, wodurch ein wasserstoffhaltiger Einsatzgasstrom erzeugbar ist;
(d) Einen Reaktor (12) konfiguriert zum Umsetzen des wasserstoffhaltigen Einsatzgasstrom unter Beteiligung von Sauerstoff als Oxidationsmittel in einem Reformierungsschritt, wodurch ein Synthesegasstrom erzeugbar ist.

**15.** Anlage nach Anspruch 14, **dadurch gekennzeichnet, dass** der Reaktor (d) ein autothermer Reformer ist oder ein für eine partielle Oxidation konfigurierter Reaktor ist.

**16.** Anlage nach Anspruch 14 oder 15, **dadurch gekennzeichnet, dass** die Mittel (c) so konfiguriert sind, dass eine

zum kohlenwasserstoffhaltigen Einsatzgasstrom zuführbare Menge an elektrolytisch erzeugtem Wasserstoffstrom so einstellbar ist, dass gemäß (d) ein Synthesegasstrom erzeugbar ist, welcher eine Stöchiometriezahl SN von 1,9 bis 2,5 aufweist, vorzugsweise von 2,0 bis 2,4 aufweist, wobei gilt

$$SN = \frac{n(H_2) - n(CO_2)}{n(CO) + n(CO_2)}, mit\ n\ in\ [mol].$$

**17.** Anlage nach einem der Ansprüche 14 bis 16, **dadurch gekennzeichnet, dass** die Anlage keine Mittel zum Entfernen von als Verunreinigung auftretendem Sauerstoff aus dem elektrolytisch erzeugbaren Wasserstoffstrom beinhaltet.

**18.** Anlage nach einem der Ansprüche 14 bis 17, **dadurch gekennzeichnet, dass** der Elektrolyseur (18) zum Bereitstellen eines elektrolytisch erzeugten Sauerstoffstroms konfiguriert ist, und die Anlage Mittel zum Verwenden des Sauerstoffstroms als Oxidationsmittel im Reaktor (d) (12) zum Umsetzen des wasserstoffhaltigen Einsatzgasstrom zum Synthesegasstrom umfasst.

**19.** Anlage nach einem der Ansprüche 14 bis 17, **dadurch gekennzeichnet, dass** die Anlage eine Vorrichtung zur Luftzerlegung umfasst, wobei durch die Vorrichtung zur Luftzerlegung ein Sauerstoffstrom erzeugbar ist, und die Anlage Mittel zum Verwenden des Sauerstoffstroms als Oxidationsmittel im Reaktor zum Umsetzen des wasserstoffhaltigen Einsatzgasstrom zum Synthesegasstrom umfasst.

**20.** Methanolanlage zur Herstellung von Methanol, umfassend eine Anlage zur Herstellung von Synthesegas nach einem der Ansprüche 14 bis 19, **dadurch gekennzeichnet, dass** die Methanolanlage einen Methanolsynthese-Reaktor (15) aufweist, wobei der Methanolsynthese-Reaktor zum Umsetzen des durch die Anlage erzeugbaren Synthesegases zu Rohmethanol konfiguriert ist, wobei das Rohmethanol zumindest Methanol ($CH_3OH$) und Wasser enthält.

**21.** Methanolanlage nach Anspruch 20, **dadurch gekennzeichnet, dass** die Methanolanlage eine thermische Trennvorrichtung (16) aufweist, wobei die thermische Trennvorrichtung zum Auftrennen des Rohmethanols in Reinmethanol und Wasser konfiguriert ist.

**22.** Methanolanlage nach Anspruch 21, **dadurch gekennzeichnet, dass** die Methanolanlage Mittel zum Verwenden des abgetrennten Wassers als Ausgangsmaterial für den elektrolytisch erzeugbaren Wasserstoff umfasst.

**23.** Methanolanlage nach einem der Ansprüche 20 bis 22, **dadurch gekennzeichnet, dass** durch das Umsetzen des durch die Anlage erzeugbaren Synthesegases zu Rohmethanol ein Restgasstrom erzeugbar ist, wobei der Restgasstrom nicht zu Rohmethanol umgesetztes Synthesegas enthält, und die Methanolanlage Mittel zum Abtrennen eines Spülgasstroms von dem Restgasstrom aufweist, und die Methanolanlage eine Wasserstoffrückgewinnungsvorrichtung aufweist, und Mittel zum Zuführen des Spülgasstroms zu der Wasserstoffrückgewinnungsvorrichtung (9) aufweist, wodurch ein nicht elektrolytisch erzeugter Wasserstoffstrom mittels der Wasserstoffrückgewinnungsvorrichtung aus dem Spülgasstrom erzeugbar ist, und die Methanolanlage

- Mittel zum Zuführen zumindest eines Teils des nicht elektrolytisch erzeugten Wasserstoffstroms zusätzlich zu dem elektrolytisch erzeugten Wasserstoffstrom zu dem kohlenwasserstoffhaltigen Einsatzgasstrom aufweist, wodurch der wasserstoffhaltige Einsatzgasstrom gemäß (c) erzeugbar ist und/oder
- Mittel zum Zuführen zumindest eines Teils des nicht elektrolytisch erzeugten Wasserstoffstroms zu dem gemäß (d) erzeugbaren Synthesegasstrom aufweist.

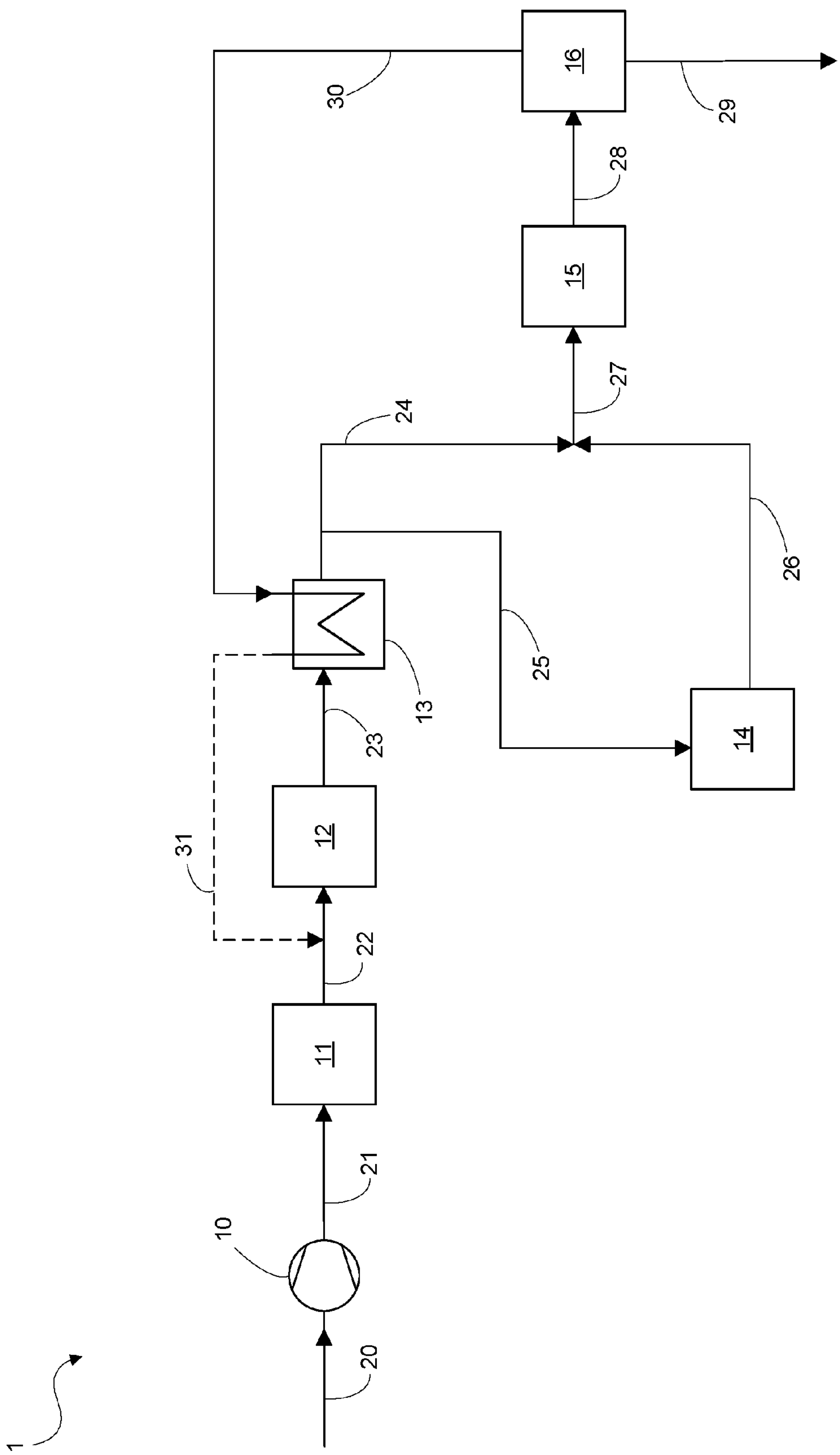


Fig. 1

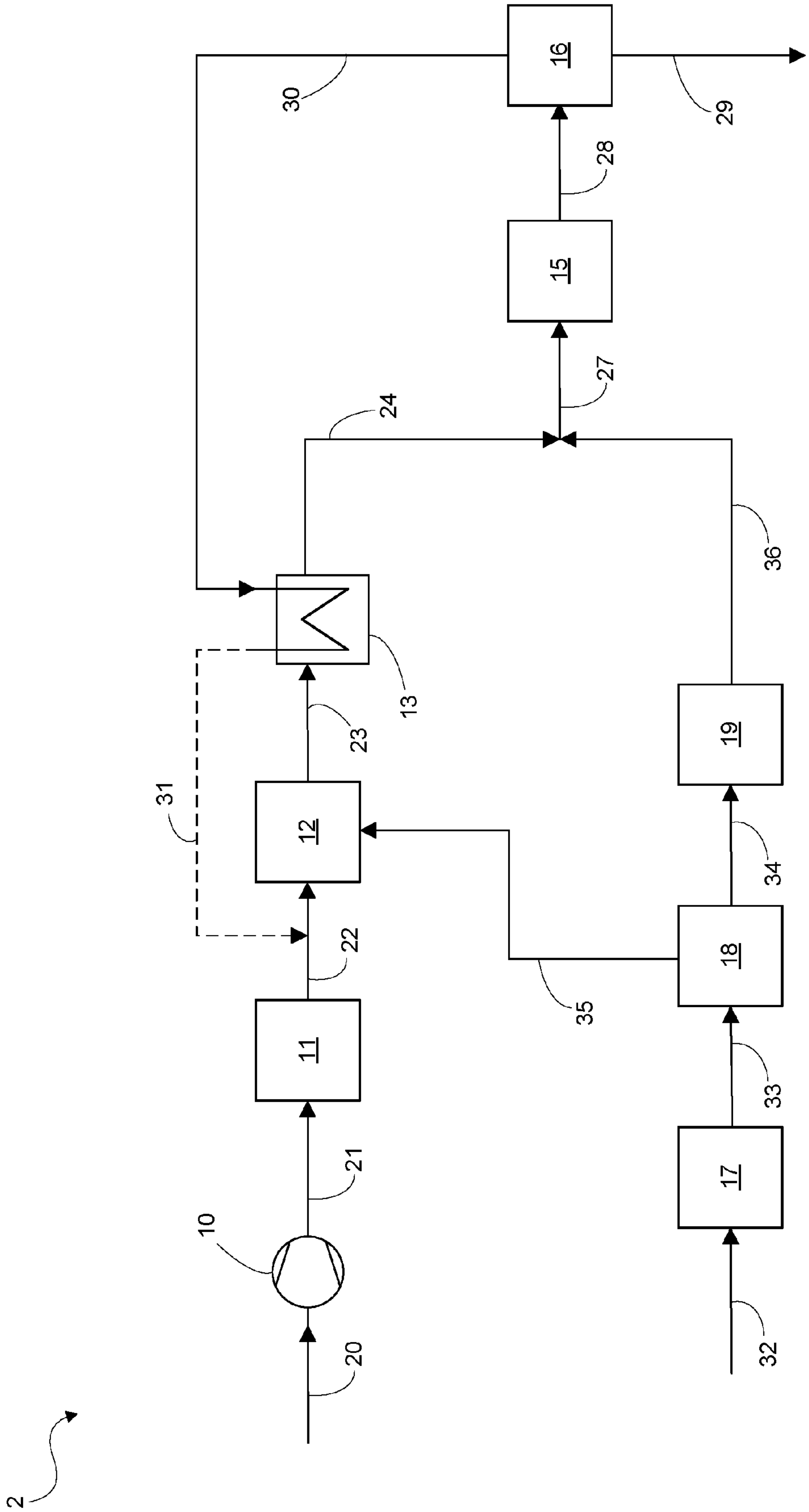


Fig. 2

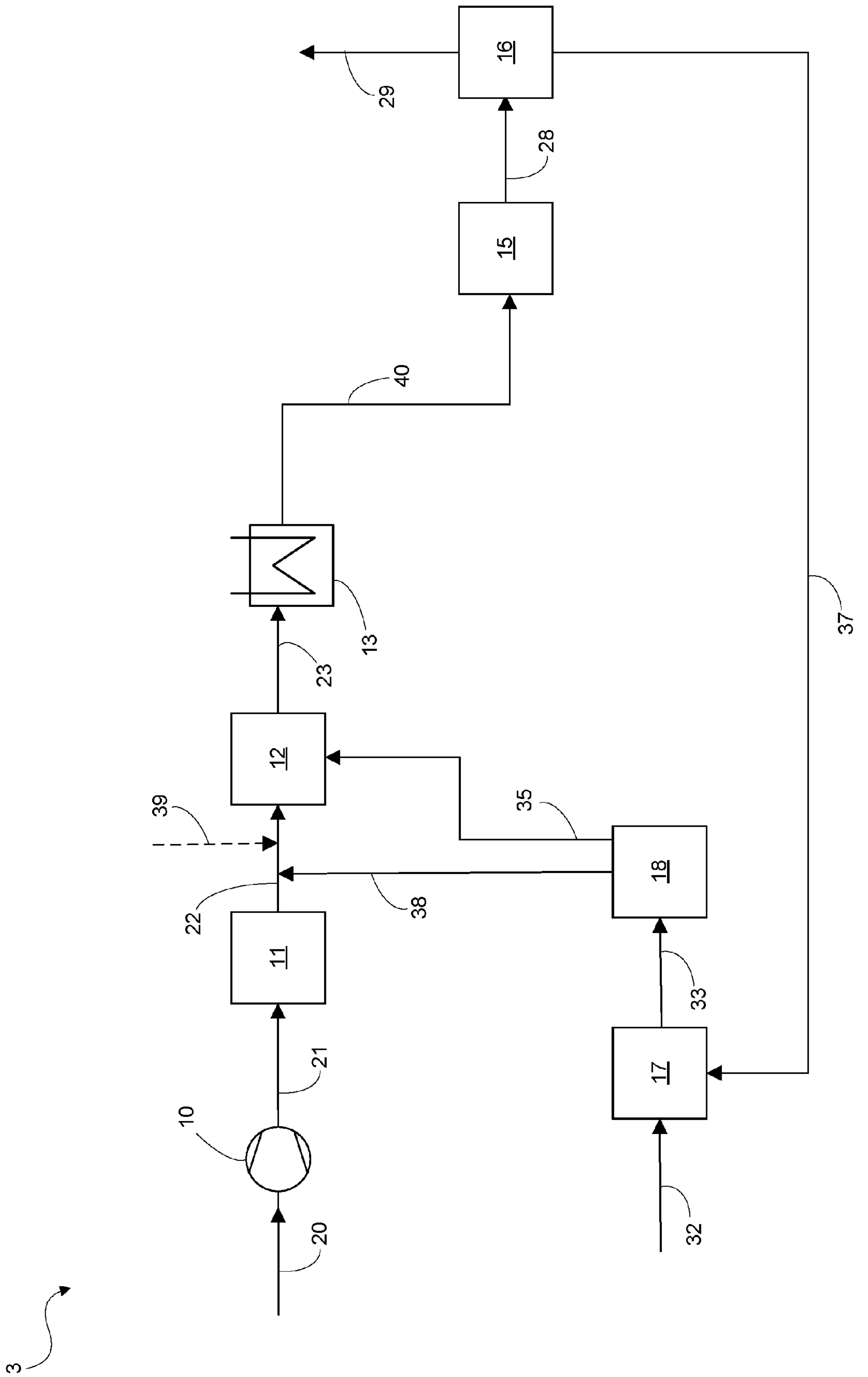


Fig. 3

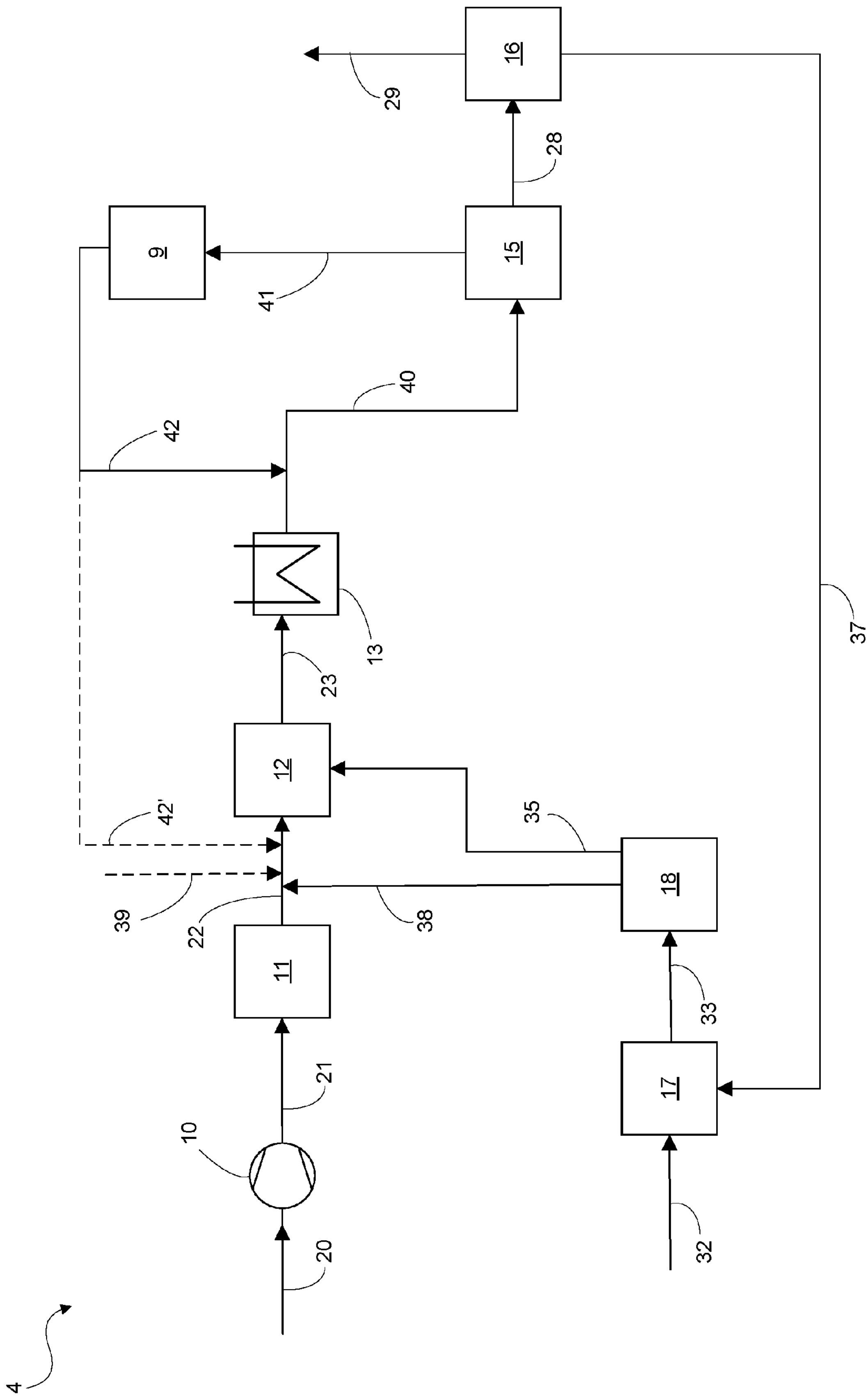

4
20
10
21
11
22
39
42'
38
12
35
23
13
40
42
41
9
15
28
16
29
18
33
17
32
37


Fig. 4

Europäisches Patentamt
European Patent Office
Office européen des brevets

# EUROPÄISCHER RECHERCHENBERICHT

Nummer der Anmeldung
EP 22 19 0457

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y<br>A | WO 2022/079002 A1 (HALDOR TOPSOE AS [DK]) 21. April 2022 (2022-04-21)<br>* Seite 5, Zeilen 6-31; Abbildung 3; Beispiel 1 *<br>* Seite 17, Zeilen 13-15 *<br>* Seite 18, Zeilen 4-25 *<br>* Seite 21, Zeile 23 - Seite 22, Zeile 14 *<br>----- | 1-10, 14-20<br>11,21<br>12,13, 22,23 | INV.<br>C01B3/36<br>C01B3/38<br>C07C29/151<br>C25B1/04 |
| Y<br>A | US 5 998 489 A (KOBAYASHI KAZUTO [JP] ET AL) 7. Dezember 1999 (1999-12-07)<br>* Anspruch 1 *<br>----- | 11,21<br>1-10, 12-20, 22,23 | |
| X<br>A | WO 2022/079010 A1 (HALDOR TOPSOE AS [DK]) 21. April 2022 (2022-04-21)<br>* Seite 21, Zeile 30 - Seite 22, Zeile 9; Abbildung 1 *<br>* Seite 23, Zeilen 5-19,22-27 *<br>* Seite 24, Zeilen 24-25 *<br>* Seite 26, Zeilen 3-32 *<br>----- | 1-10, 14-18,20<br>11-13, 19,21-23 | **RECHERCHIERTE SACHGEBIETE (IPC)** |
| A,D | EP 3 658 494 B1 (HALDOR TOPSOE AS [DK]) 19. Januar 2022 (2022-01-19)<br>* Ansprüche 1,9 *<br>----- | 1-23 | C01B<br>C07C<br>C25B |

Der vorliegende Recherchenbericht wurde für alle Patentansprüche erstellt

1

| Recherchenort | Abschlußdatum der Recherche | Prüfer |
|---|---|---|
| München | 23. Januar 2023 | Werner, Håkan |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

# ANHANG ZUM EUROPÄISCHEN RECHERCHENBERICHT ÜBER DIE EUROPÄISCHE PATENTANMELDUNG NR.

EP 22 19 0457

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-01-2023

| Im Recherchenbericht angeführtes Patentdokument | Datum der Veröffentlichung | Mitglied(er) der Patentfamilie | Datum der Veröffentlichung |
|---|---|---|---|
| WO 2022079002 A1 | 21-04-2022 | KEINE | |
| US 5998489 A | 07-12-1999 | DE 69811870 T2 | 16-10-2003 |
| | | DK 0970939 T3 | 07-04-2003 |
| | | EP 0970939 A1 | 12-01-2000 |
| | | JP 3848716 B2 | 22-11-2006 |
| | | JP H10204008 A | 04-08-1998 |
| | | US 5998489 A | 07-12-1999 |
| WO 2022079010 A1 | 21-04-2022 | KEINE | |
| EP 3658494 B1 | 19-01-2022 | AU 2018305876 A1 | 23-01-2020 |
| | | BR 112020001502 A2 | 21-07-2020 |
| | | CA 3069614 A1 | 31-01-2019 |
| | | CL 2020000157 A1 | 31-07-2020 |
| | | CN 110799452 A | 14-02-2020 |
| | | EP 3658494 A1 | 03-06-2020 |
| | | IL 271938 A | 27-02-2020 |
| | | KR 20200031633 A | 24-03-2020 |
| | | PE 20200651 A1 | 11-06-2020 |
| | | US 2020131034 A1 | 30-04-2020 |
| | | WO 2019020513 A1 | 31-01-2019 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 3658494 B1